Europäisches Patentamt

European Patent Office

Office européen des brevets

(11) Publication number: **0 311 310 B1**

(12) **EUROPEAN PATENT SPECIFICATION**

(45) Date of publication of patent specification: **20.05.92**  (51) Int. Cl.⁵: **C07C 2/12**, C10G 71/04, C07C 309/25, //B01J29/28

(21) Application number: **88309083.9**

(22) Date of filing: **30.09.88**

(54) **Olefin oligomerization.**

(30) Priority: **07.10.87 US 105438**
**07.10.87 US 105434**
**04.01.88 US 140361**

(43) Date of publication of application:
**12.04.89 Bulletin 89/15**

(45) Publication of the grant of the patent:
**20.05.92 Bulletin 92/21**

(84) Designated Contracting States:
**BE DE FR GB IT NL**

(56) References cited:
CA-A- 1 205 792     DE-A- 2 347 235
US-A- 4 104 151     US-A- 4 298 547
US-A- 4 520 221     US-A- 4 568 786
US-A- 4 658 079

(73) Proprietor: **MOBIL OIL CORPORATION**
**150 East 42nd Street**
**New York New York 10017(US)**

(72) Inventor: **Blain, David Alan**
**642-A Rose Hollow Drive**
**Yardley Pennsylvania 19067(US)**
Inventor: **Page, Nancy Marie**
**289 Flint Court**
**Yardley Pennsylvania 19067(US)**
Inventor: **Young, Lewis Brewster**
**26 Pineview Court**
**Skillman New Jersey 08558(US)**

(74) Representative: **Colmer, Stephen Gary**
**Mobil Court 3 Clements Inn**
**London WC2A 2EB(GB)**

# EP 0 311 310 B1

## Description

This invention relates to a process for oligomerizing a lower olefin feedstock to produce substantially linear hydrocarbons of higher molecular weight than the feedstock.

It is well known that lower olefins can be oligomerized to higher molecular weight olefinic hydrocarbons by passage over a zeolite catalyst. For example, U.S. Patent No. 3960978 discloses the conversion of $C_2$ - $C_5$ olefins, either alone or in admixture with paraffins, into an olefinic gasoline blending stock by contacting the olefins at a temperature of 260 - 480°C (500 - 900°F) with ZSM-5 or ZSM-11. More recent advances in this process are disclosed in U.S. Patent Nos. 4,150,062, 4,211,640, 4,227,992 and 4,547,613.

It is also known that by controlling the conditions of olefin oligomerization over a medium pore, shape selective zeolite, such as ZSM-5, it is possible to favor the formation of hydrocarbons of a particular molecular weight distribution. For example, as reported by Garwood in "Intrazeolite Chemistry 23", (Amer. Chem. Soc., 1983), reaction conditions favoring higher molecular weight products, such as lubricant range hydrocarbons, are low temperature (200° - 260°C.), elevated pressure (about 2000 kPa or greater), and long contact time (less than 1 WHSV).

One factor which is frequently of particular importance in olefin oligomerization is the molecular conformation of the product. Thus, where the product is intended for use as a lubricating oil, it is well known that extensive branching is undesirable since it tends to lower the viscosity index of the lubricant. Another end use where linearity in the oligomerized product is important is in the production of aklylbenzene sulfonic acid based detergents. Such detergents can be produced by alkylating benzene with a long-chain olefin and then sulfonating the product to produce a detergent whose rate of biodegradability is dependent on the linearity of the olefinic starting material.

It is known from, for example, U.S. Patent Nos. 4,520,221 and 4,568,786 that when effecting olefin oligomerization over a medium pore, shape-selective zeolite catalyst, such as ZSM-5, lubricant range hydrocarbons of increased linearity can be produced by modifying the zolite with a bulky amine to reduce its surface activity. Similar results are obtained according to U.S. Patent No. 4,658,079 when the surface of ZSM-5 is modified with a bulky organophosphorus compound.

Despite these advances; there is a continuing need to improve the yield of linear hydrocarbons produced by olefin oligomerization and an object of the present invention is to provide such an improvement.

Accordingly, the invention resides in a process for oligomerizing an olefinic feedstock comprising the step contacting the feedstock with ZSN-23 which is surface deactivated with a trialkylpyridine compound having an effective cross-section larger than that of the pores of ZSM-23.

Preferably the alkyl groups of the trialkylpyridine compound have 1 - 4 carbon atoms and more preferably are in the 2, 4, 6 positions. Most preferably, the compound is 2, 4, 6 collidine.

By employing a surface-inactivated, but internally active, ZSM-23 zeolite catalyst in olefin oligomerization, the process of the invention yields a high quality, essentially linear oligomer stock which can, for example, be efficiently converted to high VI lube oils. The catalyst can be surface inactivated in situ by cofeeding the sterically hindered trialkyl pyridine compound or the organophosphite compound with the olefinic feedstock, or the catalyst can be treated in a separate step prior to olefin oligomerization.

ZSM-23 is an intermediate pore size zeolite, which may be synthesized with Brönsted acid active sites by incorporating a tetrahedrally coordinated metal, such as Al, Ga, or Fe, within the silicate framework. ZSM-23 has a crystalline structure which is readily recognized by its X-ray diffraction pattern and which is described in U.S. Patent 4,076,842. When used in the process of the invention, ZSM-23 preferably has a crystallite size of 0.02 to 2 microns and is combined with a metal oxide binder, preferably an alumina binder in the form of cylindrical extrudates of 1-5 mm in length. As shown in the Examples, improved results may also be obtained if the ZSM-23 catalyst is steamed prior to use in the present invention, suitable steaming conditions comprising heating in a steam-containing atmosphere at 250-950°C, preferably 400-650°C, for 1/2 - 100 hours, preferably 1 - 24 hours.

At least part of the surface Brönsted acid sites of the ZSM-23 are neutralized by chemisorption of a surface deactivating agent and generally it may be desirable to neutralize more than 15% of the total Brönsted acid sites by chemisorption of the surface deactivating agent. In one embodiment, the surface deactivating agent employed is a trialkyl pyridine species having alkyl groups of 1 to 4 carbon atoms, preferably at the 2, 4 and 6 positions. The selected organonitrogen compounds have an effective cross section larger than that of the pores of ZSM-23 so as to prevent their infusion into the internal pores of the catalyst, but are insufficiently large that steric hindrance prevents effective interaction with surface Brönsted acid sites. Preferably the surface deactivating agent is 2, 4, 6-collidine (2, 4, 6-trimethyl pyridine, gamma-collidine).

2

According to the process of the invention an olefin feedstock normally comprising $C_2$-$C_{10}$ olefins, preferably $C_3$-$C_6$ olefins, more preferably $C_3$-$C_4$ olefins and most preferably propylene is contacted with ZSM-23 which has been surface deactivated in a prior treatment step or is surface deactivated by co-feeding a bulky organonitrogen compound as described above. Preferably, a combination of a prior treatment with, and co-feeding of, the surface deactivating agent is employed. The temperature used to effect oligomerization is typically 100 - 350°C, or more preferably 150 - 250°C, since below 150°C conversion is slow and above 250°C there is a tendency for cracking of the oligomerized product to occur. Other conditions include a pressure of atmospheric to 20,000 kPa, preferably 1000 to 10,000 kPa, and a WHSV of 0.01 - 2, preferably 0.2 - 1.0.

When propylene or butene are oligomerized according to the present process, a unique mixture of liquid hydrocarbon products is formed. More particularly, this mixture of hydrocarbons may comprise at least 95% by weight of mono-olefins oligomers of the empirical formula

$$C_{(n + nm)}H_{2(n + nm)}$$

where n is 3 or 4 and m is an integer from 1 to 6, said mono-olefin oligomers comprising at least 20 percent by weight of olefins having at least 12 carbon atoms, said olefins having at least 12 carbon atoms having an average of from 0.80 to 2.00 methyl side groups per carbon chain, said olefins not having any side groups other than methyl.

It will be understood that methyl side groups are methyl groups which occupy positions other than the terminal positions of the first and last (i.e., alpha and omega) carbon atoms of the longest carbon chain. This longest carbon chain is also referred to herein as the straight backbone chain of the olefin. The average number of methyl side groups for the $C_{12+}$ olefins will generally be within the range 0.80 to 2.00.

These oligomers may be separated into fractions by conventional distillation separation. When propylene is oligomerized olefin fractions containing the following numbers of carbon atoms can be obtained: 6, 9, 12, 15, 18 and 21. When butene is oligomerized, olefin fractions containing the following numbers of carbon atoms my be obtained: 8, 12, 16, 20, 24 and 28. It is also possible to oligomerize a mixture of propylene and butene and to obtain a mixture of oligomers having at least 6 carbon atoms.

Thus by fractionating an oligomerization product prepared by the present process, it is possible to obtain a mixture of hydrocarbons comprising at least 95 (e.g., at least 98) percent by weight of mono-olefins having 12 carbon atoms, said mono-olefins having a straight backbone chain of at least 10 carbon atoms, and an average of 0.40 to 2.00 methyl side groups per carbon chain. These $C_{12}$ olefins conveniently comprise at least 5, e.g. 5 to 40, mole percent dodecene, at least 30, e.g., 30 to 90, mole percent methylundecene and at least 5, e.g., from 5 to 40, mole percent dimethyldecene. In addition $C_{15}$, $C_{16}$ and $C_{18}$ olefin fractions having similar compositions can be isolated.

These olefin mixtures, particularly the above-mentioned fractionation products, may be used in the production of high viscosity index lubricating oils. This is conveniently effected by passing all or a portion (preferably the $C_{12+}$ fraction) of the oligomerization product over a $BF_3/H_3PO_4$ catalyst, or more preferably a ZSM-5 catalyst, to effect further oligomerization, typically to a $C_{25}+$ product. Another use for the oligomerization products of the invention, particularly the $C_{12}$ fractions, is as alkylating agents in a process for the selective alkylation of an aromatic compound (e.g., benzene) with a relatively long chain length alkylating agent to produce substantially linear phenylalkanes. Catalysts and reaction conditions for this alkylation process are disclosed in the aforementioned U.S. Patent No. 4,301,317, which describes a similar alkylation process using linear olefins (e.g., dodecene) as alkylating agents.

The alkylation process may be carried out by contacting the aromatic compound with the alkylating agent in the presence of a zeolite catalyst under suitable alkylation conditions including a temperature of 50 to 500°C, more preferably 100 to 350°C, a pressure of $2.5 \times 10^4$ $N/m^2$ to $2.5 \ 10^7$ $N/m^2$ (0.25-250 atmospheres), more preferably $10^5$ to $5 \times 10^6$ $N/m^2$, and a WHSV of 0.1 to 500, more preferably 0.5 to 100.

The aromatic compounds employed are benzene compounds, which may be unsubstituted, or may carry from 1 to 2 substituents on the ring structure. If substituted, the substituent may be an alkyl group having from 1 to 10 carbon atoms or may be a halide alkoxy, or aryl group or any combination of such substituents.

The zeolites utilized in the alkylation process may be either naturally occuring or synthetic and include, by way of example, cancrinite, gmelinite, mordenite, dealuminized mordenite and offretite. Also contemplated are synthetic and naturally occuring isotypes of such zeolite materials, such as: zeolite S, zeolite NaS, zeolite NaD, Ptilolite, Zeolon, zeolite O, and TMA-offretite. Dealuminized mordenite is the preferred catalyst.

The zeolites used in the alkylation process will generally have at least 10 percent of the cationic sites thereof occupied by ions other than alkali or alkaline-earth metals. Typical replacing ions include ammonium, hydrogen, rare earth, zinc, copper and aluminum. The zeolites may desirably be subjected prior to use to thermal treatment, including steaming or calcination in air, hydrogen or an inert gas, e.g. nitrogen or helium. In addition, the zeolites may be bound in conventional manner with inorganic materials such as clay, silica, and/or metal oxides.

The above-discussed alkylation process is highly selective to the production of 2-phenylalkanes which are useful as intermediates for the production of alkylphensulfonates, the latter being useful as biodegradable detergents or surfactants. Processes for sulfonating alklbenzenes are well known and are described in, for example, U.S. Patent No. 4,298,547.

The invention will now be more particularly described with reference to the following Examples.

EXAMPLE I (Comparative)

Aluminosilicate H-ZSM-23 extrudate (65% zeolite, 35% alumina binder) was loaded into a metal pressurized reactor and calcined overnight at 500°C. The catalyst was then used to oligomerize propylene to intermediate molecular weight olefins at various temperatures and feed rates. These results are summarized in Table 1.

TABLE 1

| Run No. | $C_3$ = WHSV | Temp. °C | $C_{12+}$ Select. wt.%[a] | Branching Index[b] | Branching Methyls[b] per $C_{15}$ |
|---------|--------------|----------|--------------------------|---------------------|-----------------------------------|
| 1-A | 1.0 | 160 | 61.5 | 51.7 | 3.5 |
| 1-B | 0.5 | 160 | 73.7 | 51.4 | 3.5 |
| 1-C | 0.5 | 200 | 78.6 | 54.7 | 3.8 |
| 1-D | 1.0 | 200 | 81.7 | 55.1 | 3.9 |
| 1-E | 1.0 | 225 | 78.5 | 52.3 | 3.6 |

(a) In crude reaction product
(b) In crude $C_{12+}$ fraction

The determination of Branching Index is a useful and sensitive method for quantitatively assessing the degree of linearity of a molecule or molecular mixture. The index is determined as follows: the C6 and C9 oligomers are first removed from the sample and the C12+ fraction is hydrogenated using Pd/charcoal catalyst in acetic acid. The hydrogenated sample is extracted from the acetic acid into deutrochloroform and the $^1$H NMR spectrum determined. The branching index is defined as the ratio of the intensity (area) of the resonance due to CH3 (0.7-1.0 ppm) divided by the sum of the intensities (areas) of the resonances due to CH3 (0.7.-1.0 ppm) and CH2 (1.1-1.8 ppm). The number of methyl groups per molecule is defined by the equation

$$Me/molecule = B.I.*(n+1))/150$$

where
B. I. =    branching index as defined above and
n =    carbon number of the fraction of interest.

This calculated number of methyls per molecule includes the two terminal methyl groups. Therefore, to determine the actual number of methyl side groups, e.g.. mid-chain methyl groups, these two groups must be subtracted from the total methyl/molecule value calculated.

EXAMPLE II (Comparative)

The catalyst used in Example I was calcined in a reactor overnight at 500°C. The calcined catalyst was then cooled to room temperature in the reactor, and a solution containing 1 gram 2,6-di-t-butyl pyridine per 100 ml pentane was passed over the catalyst until a total of 6 ml of deactivating solution per gram of catalyst had been used. Following this treatment, the catalyst was purged with nitrogen for one hour at room temperature, then the reactor temperature was slowly increased and oligomerization of propylene was initiated. During the reaction, a small amount of 2,6-di-t-butyl pyridine (DTBP) solution was co-fed to maintain surface deactivation. The results are summarized in Table 2.

TABLE 2

| Run No. | $C_3 =$ WHSV | 2,6- DTBP ppm | Temp. ° C. | $C_{12+}$ Select wt.%[a] | Branching Index[b] | Branching Methyls[b] per $C_{15}$ |
|---|---|---|---|---|---|---|
| 2-A | 1.0 | 400 | 175 | 18.4 | 40.0 | 2.3 |
| 2-B | 0.5 | 800 | 200 | 43.8 | 39.4 | 2.2 |
| 2-C | 1.0 | 400 | 200 | 32.2 | 40.7 | 2.3 |
| 2-D | 0.5 | 800 | 200 | 50.4 | 41.9 | 2.5 |

(a) In crude reaction product
(b) In crude $C_{12+}$ fraction

## EXAMPLE III

H-ZSM-23, prepared as in Example I was treated with deactivating solution as in Example II, except that the basic component was 2,4,6-collidine. A small co-feed of 2,4,6-collidine solution was continued during reaction to maintain surface deactivation. Results of these reactions are summarized in Table 3.

TABLE 3

| Run No. | $C_3 =$ WHSV | 2,4,6- Coll., ppm | Temp. ° C. | $C_{12+}$ Select. wt.%[a] | Branching Index[b] | Branching Methyls[b] per $C_{15}$ |
|---|---|---|---|---|---|---|
| 3-A | 0.5 | 200 | 200 | 24.7 | 35.5 | 1.8 |
| 3-B | 0.25 | 400 | 200 | 35.1 | 34.9 | 1.7 |
| 3-C | 0.25 | 400 | 212 | 39.7 | 37.2 | 2.0 |
| 3-D | 0.25 | 400 | 225 | 33.5 | 37.6 | 2.0 |
| 3-E | 0.25 | 200 | 225 | 36.4 | 40.4 | 2.3 |

(a) In crude reaction product
(b) In crude $C_{12}$ + fraction

The above experimental runs were conducted at a pressure of about 3500-4300kPa (500-600 psig.). Comparison of examples run at equivalent space velocity and temperature (e.g., 0.5 WHSV and 200°C.) show significant improvement in product linearity employing the trialkylpyridine surface deactivating agent.

## EXAMPLE IV

Propylene was contacted according to the procedure of Example I with 2,4,6-collidine modified HZSM-23 in a flow reactor at 200°C at the rate of 0.25 g propylene/g zeolite/hr. The crude product was distilled to obtain a $C_{15}^+$ fraction. The $C_{15}^+$ fraction was contacted with $BF_3$/70% aqueous phosphoric acid catalyst at room temperature for about 4 hours. The crude product, containing about 75 wt% of $C_{25}^+$ lube range hydrocarbon was stripped to remove the $C_{24}^-$ hydrocarbons. The viscosity index of the $C_{25}^+$ fraction was 128; the 100°C viscosity was 8.2 cSt.

## EXAMPLE V (Comparative)

15.4 gms HZSM.5 (65% zeolite, 35% alumina binder) were treated with 0.18 grams 2,4,6-collidine in approximately 50 cc pentane. This represents 0.25 moles amine per mole of acid in the zeolite. The pentane was allowed to evaporate at room temperature and the surface modified catalyst charged to a fixed bed tubular reactor at superatmospheric pressure. Propylene was metered to the reactor and a solution of 1 gram 2,4,6-collidine in 500 ml pentane was also metered to the reactor to give approximately 0.2 mmoles amine per mole H + in the zeolite per hour. Reaction temperature was adjusted to achieve approximately 50% propylene conversion

| TEMP | 205°C |
|---|---|
| PRESSURE | 3600 kPa (500 psig) |
| C3 = WHSV, HR-1 | 0.21 |
| DEACTIVATING AGENT IN FEED | 65 ppm |
| C3 = CONV, WT% | 55.0 |
| C12 + SELECTIVITY | 20.1% |
| C15 + | 5.9 |
| BRANCHING INDEX | 32.8 |
| BRANCHING METHYLS PER C15 | 1.5 |

EXAMPLE VI (Comparative)

Example V was repeated, except that 15.4 gms ZSM-5 (65% zeolite, 35% alumina binder) are treated with a solution containing 0.28 grams, 2,6-di-t-butylpyridine in pentane. (0.25 moles amine per mole H + in the zeolite). Comparative results are summarized as follows:

| TEMP | 145°C |
|---|---|
| PRESSURE | 3600 kPa |
| C3 = WHSV, HR-1 | 0.22 |
| AMINE IN FEED | 100 ppm |
| C3 = CONVERSION, WT% | 59.1 |
| C12 + SELECTIVITY | 9.8 |
| BRANCHING INDEX | 38.4 |
| BRANCHING METHYLS PER C15 | 2.1 |

EXAMPLE VII

Example V was repeated, except 15.4 gms HZSM 23 (65% zeolite, 35% alumina binder) was treated with 0.088 gms, 2,4,6-collidine in approximately 50 ml pentane. (0.25 moles amine per mole H + in the zeolite.) The conditions were adjusted to achieve approximately 50% propylene conversion. The results are summarized as follows:

| TEMP | 175°C |
|---|---|
| PRESSURE | 3600 kPa |
| C3 = WHSV, HR-1 | 0.21 |
| AMINE IN FEED | 200 ppm |
| C3 = CONVERSION, WT% | 57.7 |
| C12 + SELECTIVITY | 22.0 |
| BRANCHING INDEX | 30.5 |
| BRANCHING METHYLS PER C15 | 1.25 |

EXAMPLE VIII (Comparative)

Example VII was repeated, except 15.4 gms ZSM-23 (65% zeolite, 35% alumina binder) were treated with 0.14 gms, 2,6-di-t-butylpyridine in approximately 50 ml pentane. (0.55 moles amine per mole H + in the zeolite.) Results are summarized as follows:

| TEMP | 145°C |
|---|---|
| PRESSURE | 3600 kPa |
| C3 = WHSV, HR-1 | 0.21 |
| AMINE IN FEED | 50 ppm |
| C3 = CONVERSION, WT% | 59.0 |
| C12+ SELECTIVITY | 21.6 |
| BRANCHING INDEX | 31.4 |
| BRANCHING METHYLS PER C15 | 1.35 |

## EXAMPLE IX

A run of extended duration was carried out in a stirred one gallon autoclave. In accordance with this run, sufficient propylene was charged to the reactor to give the desired reaction pressure at temperature. Propylene was fed on demand during the reaction to maintain this pressure. When enough liquid product had been made to fill the autoclave to approximately one-half of capacity, the product was discharged through a dip tube. Additional propylene was then charged to give the desired pressure and the reaction resumed. This run lasted for slightly longer than two months. No amine (i.e. 2, 4, 6-collidine) was used as a cofeed during the entire duration of this run.

In this run, 125 gms. HZSM-23/alumina extrudate were treated with 1.5 gms. 2, 4, 6-collidine and the catalyst charged to the autoclave. The HZSM-23 had a silica to alumina ratio of approximately 110 and an alpha value of about 25. This run continued for a total of 81 days producing approximately 85 kg crude propylene oligomer. The temperature of the reaction was constantly maintained at 225°C and the pressure of the autoclave during the reactions was constantly maintained at 5270 kPa (750 psig). The selectivity to $C_{12+}$ product varied essentially randomly between about 30 and 40 wt.% of the total product. A plot of this $C_{12+}$ product selectivity indicated that the average selectivity to $C_{12+}$ product was essentially constant over the 81 day duration of the run. The average number of methyl side groups in the $C_{12}$ product increased very gradually from slightly less than about 1.20 to about 1.35 over the duration of the run, with the highest level of methyl branching per $C_{12}$ molecule (e.g., 1.35-1.40) being observed when the $C_{12+}$ product selectivity was relatively high (e.g., at about 40 wt.%).

## EXAMPLE X

This Example provides data regarding the use of a mixed feed of propane and propylene. An HZSM-23 extrudate (65% zeolite, 35% alumina binder) was treated with a solution of 2, 4, 6-collidine in pentane, and the pentane was permitted to evaporate at room temperature. This HZSM-23 had a silica to alumina ratio of approximately 110 and an alpha value of about 25. This catalyst was charged to a fixed bed tubular reactor. In Runs 13-A and 13-B a synthetic propane/propylene (P/P) feed (60% propylene/40% propane) was fed into the reactor. In Run 13-C propylene in the absence of a propane diluent was fed into the reactor. 2, 4, 6-Collidine (i.e., "Poison") was also fed into the reactor in varying amounts. Product distributions and branching observed at two reaction pressures with the synthetic P/P mix and with pure propylene feed are shown in Table 4.

EP 0 311 310 B1

Table 4

| COMPARISON OF PROPANE/PROPYLENE VS PURE PROPYLENE FEEDS | | | |
|---|---|---|---|
| Run Number | 13-A | 13-B | 13-C |
| Feed | P/P | P/P | C |
| Temp (setting) | 185 | 185 | 185 |
| Temp (hot spot) | 212 | 217 | 214 |
| Pressure, psig (kPa) | 500 (3550) | 800 (5620) | 500 (3550) |
| C WHSV, $hr^{-1}$ | 0.21 | 0.19 | 0.24 |
| $C_3$ WHSV, $hr^{-1}$ | 0.15 | 0.13 | 0.00 |
| Poison, ppm | 92 | 83 | 200 |
| $C_3^=$ Conv, wt.% | 74.6 | 74.3 | 91.1 |
| Prod Dist, wt.% | | | |
| $C_6$ | 40.8 | 51.4 | 58.7 |
| $C_9$ | 24.4 | 23.5 | 20.1 |
| $C_{12}$ | 13.6 | 11.9 | 11.0 |
| $C_{15}$ | 11.8 | 7.8 | 6.4 |
| $C_{18}$ | 5.7 | 3.2 | 2.4 |
| $C_{21}^+$ | 3.8 | 2.1 | 1.4 |
| $C_{12}$ + Select | 34.9 | 25.0 | 21.2 |
| Branching | | | |
| $Me/C_9$ | 1.36 | 1.08 | 1.05 |
| $Me/C_{12}$ | 1.68 | 1.27 | 1.19 |
| $Me/C_{15}$ | 1.85 | 1.39 | 1.28 |

Runs 13-A and 13C were at the same total reactor pressure (500 psig) while Runs 13-B and 13-C were at essentially the same propylene partial pressure. The slightly higher linearity and 1lower $C_{12+}$ selectivity observed with pure propylene at 500 psig versus P/P mix at 800 psig (480 psig $C_3^=$) may be due in part to the higher poison level in the former run. It can be seen that the two runs at the same propylene partial pressure gave very similar product distributions and branching. This suggests that as diluent is added, the reaction should be run at a higher total pressure (constant $C_3^=$ partial pressure) in order to obtain the same product quality.

EXAMPLE XI

This Example provides data regarding the use of a 1-butene feedstock. 15.4 gms HZSM-23 extrudate (65% zeolite, 35% alumina binder) were treated with 0.088 grams 2, 4, 6-collidine in approximately 50 cc pentane. This HZSM-23 had a silica to alumina ratio of approximately 110 and an alpha value of about 25. The pentane was allowed to evaporate, at room temperature, and the surface modified catalyst was charged to a fixed bed tubular reactor. 1-butene was metered to the reactor, and a solution of 2, 4, 6-collidine (i.e., "Poison") in pentane was also metered to the reactor at 200 ppm of 2, 4, 6-collidine per weight of total feed

8

into the reactor. Results are summarized in Table 5.

Table 5
PRODUCT DISTRIBUTIONS OBSERVED WITH 1-BUTENE

| Run Number | 14-A | 14-B | 14-C | 14-D |
|---|---|---|---|---|
| REACTION COND | | | | |
| Temp (setting) | 180 | 185 | 195 | 205 |
| Temp (hot spot) | 217 | 224 | 236 | 249 |
| Pressure, psig (kPa) | 540 (3824) | 530 (3755) | 530 (3755) | 520 (3686) |
| $C_4^=$ WHSV, | 0.29 | 0.27 | 0.22 | 0.21 |
| Poison, ppm | 200 | 200 | 200 | 200 |
| Prod Dist | | | | |
| $C_8$ | 38.4 | 36.5 | 33.4 | 26.2 |
| $C_{12}$ | 33.1 | 34.8 | 38.5 | 43.0 |
| $C_{16}$ | 12.4 | 14.3 | 15.4 | 21.9 |
| $C_{20}$ | 4.5 | 5.1 | 3.5 | 7.0 |
| $C_{24}$ | 1.5 | 1.8 | 1.6 | 2.2 |
| $C_{28}^+$ | 1.2 | 1.4 | 0.4 | 1.4 |
| $C_{12}^+$ Select | 52.7 | 57.3 | 59.4 | 75.6 |
| $C_{16}^+$ Select | 19.6 | 22.5 | 21.0 | 32.5 |
| $C_4^=$ Conv | 28.5 | 44.3 | 69.5 | 95.4 |
| CARBON SKELETON | | | | |
| $C_8$ | | | | |
| DI-ME (a) | 6.7 | 8.6 | 11.5 | 20.1 |
| MONO-ME | 79.3 | 79.3 | 77.9 | 72.3 |
| NORMAL | 14.1 | 12.1 | 10.6 | 7.6 |
| ME/$C_8$ (b) | 0.96 | 1.01 | 1.07 | 1.23 |
| $C_{12}$ | | | | |
| DI-ME | 13.8 | 16.1 | 19.2 | 23.2 |
| MONO-ME | 78.2 | 76.7 | 74.0 | 71.1 |
| NORMAL | 8.0 | 7.3 | 6.8 | 5.7 |
| ME/$C_{12}$ | 1.13 | 1.17 | 1.22 | 1.29 |
| $C_{16}$ | | | | |
| DI-ME | 23.0 | 24.5 | 24.2 | 26.2 |
| MONO-ME | 71.5 | 70.1 | 68.4 | 68.7 |
| NORMAL | 5.5 | 5.5 | 7.4 | 5.1 |
| ME/$C_{16}$ | 1.29 | 1.31 | 1.29 | 1.34 |

9

(a) All values for isomer distributions are normalized percentages for that carbon number.

(b) Values for number of methyl branches were calculated assuming an average of 2.5 for di-methyl component.

As compared with the use of a propylene feed, much higher $C_{12+}$ selectivities were realized, but most of the $C_{12+}$ oligomers were actually $C_{12}$. There was a much more dramatic fall-off with carbon number beyond $C_{12}$ observed for 1-butene oligomerization than was observed with propylene. The isomer distributions observed for a $C_{12}$ produced from 1-butene were very similar to those observed with a propylene oligomerization product.

EXAMPLE XII

This Example illustrates the effect of steam treatment on the catalyst performance of two different ZSM-23 catalysts designated A and C respectively. Each of the unsteamed Catalysts A and C were HZSM-23 extrudates (65% zeolite, 35% alumina binder), with the HZSM-23 having a silica to alumina ratio of approximately 110 and an alpha value of about 25. Samples of each of the unsteamed catalysts A and C were steamed for four hours at 510°C (950°F) in 100% steam. Catalyst B is designated herein as the steamed counterpart of Catalyst A, and Catalyst D is designated herein as the steamed counterpart of Catalyst C.

7.7 grams of each of Catalysts A, B, C and D were treated with 0.044 grams of a solution of 2, 4, 6-collidine in pentane, and the pentane was permitted to evaporate at room temperature. Each of Catalysts A, B, C and D were charged to a 450cc stirred autoclave along with approximately 75cc propylene, and the reactor was heated to 225°C. At reaction temperature, additional propylene was added, if necessary, to give a total reaction pressure of (5272 kPa) 750 psig. Propylene was then added upon demand to maintain the pressure. Initially, all runs were to be for a total of 10 hours reaction time. However, the greater activity for the steamed catalysts necessitated termination of these reactions at short times. Results of these four runs are given in Table 6, from which it will be seen that the steam treatment resulted in a catalyst that was 3-4 times more active for propylene conversion relative to that observed for the unsteamed parent. In addition to converting propylene at a faster rate, the steamed catalysts were more selective for formation of the higher oligomers. Branching was, however, slightly higher with the steamed catalysts.

Table **6**

EFFECT OF STEAMING ON ZSM-23 PERFORMANCE

| Catalyst | A | B | C | D |
|---|---|---|---|---|
| Run Time, hrs | 10 | 5 | 10 | 3 |
| Gms liquid product | 50.1 | 102.8 | 81.3 | 64.8 |
| Rate, gms/hr | 5.0 | 20.6 | 8.1 | 21.6 |

Prod Dist. wt.%

| | A | B | C | D |
|---|---|---|---|---|
| $C_6$ | 62.3 | 49.6 | 57.3 | 52.5 |
| $C_9$ | 20.6 | 23.0 | 24.4 | 21.8 |
| $C_{12}$ | 9.8 | 12.3 | 8.7 | 10.9 |
| $C_{15}$ | 4.7 | 8.5 | 5.9 | 8.0 |
| $C_{18+}$ | 1.7 | 3.8 | 2.4 | 4.0 |
| $C_{21}$ | 0.9 | 2.7 | 1.3 | 2.9 |
| $C_{12+}$ Select | 17.1 | 27.3 | 18.3 | 25.8 |
| $C_{15+}$ Select | 7.3 | 15.0 | 9.6 | 14.9 |

Branching

| | A | B | C | D |
|---|---|---|---|---|
| $Me/C_9$ | 1.02 | 1.14 | 1.31 | 1.28 |
| $Me/C_{12}$ | 1.08 | 1.28 | 1.27 | 1.35 |
| $Me/C_{15}$ | 1.14 | 1.28 | 1.23 | 1.34 |

Catalyst A:  unsteamed
Catalyst B:  steamed A
Catalyst C:  unsteamed
Catalyst D:  steamed C

EXAMPLE XIII

This example demonstrates the use of the oligomerization product of the present process in producing alkylbenzene sulfonate detergents.

a) Dealuminized mordenite was first prepared by heating 10g of mordenite (commercially available as Zedon 100) from 50 to 400°C over 4 hours. It was kept at this temperataure for 1.5 hours, then raised to 670°C over 2 hours where it was kept for 2 hours. The resulting 8.5g catalyst was stirred for 2 hours in 170 ml 0.5 N HCl, then refluxed overnight. This acid refluxed catalyst was filtered and added to 170 ml distilled water. After refluxing overnight, the catalyst was filtered and washed with about 1500 ml distilled water. It was calcined for several hours at 568°C before use.

b) Benzene was then alkylated with an olefin mixture using the dealuminized mordenite prepared in (a). The olefin mixture was propylene tetramer (1.3 methyl branches per chain) prepared by oligomerizing propylene over a ZSM-23 catalyst having surface acid sites inactivated with 2,4,6-collidine and separating out the $C_{12}$ fraction. After percolation through activated alumina, 10 ml of the $C_{12}$ olefin (0.05 mol) were added to an oven-dried flask under $N_2$ together with 50 ml benzene (0.56 mol) and 0.6 g the dealuminized mordenite. After 6 hr., GC (gas chromatography) showed about 10% reaction. Another 0.5

EP 0 311 310 B1

g catalyst was added and the mixture was refluxed for 64 hours. GC showed about 90% reaction. Another 0.25g catalyst was added and the mixture was refluxed an additional 24 hours. The reaction mixture was cooled, filtered, and evaporated to 10.15g product, which still contained 4% unreacted olefin. All of the dodecylbenzenes were fractionally distilled with the desired product distilling at 68-111°C (mostly 90-94°C), 0.3-0.4 mmHg.

c) 5g (0.021 mol) of the dodecylbenzene prepared in (b) was then charged to a 3-neck round-bottom flask, equipped with a thermometer, and a dropping funnel. 3 ml of fuming $H_2SO_4$ (0.022 mol $SO_3$) was then added dropwise to the flask over 35 minutes, with temperature being kept below 30°C with an ice bath. The dark brown mixture was then heated to 60°C for one hour. Upon cooling, the mixture was pipetted into 40 ml 10% NaOH (aq) with vigorous stirring, causing a large amount of tan precipitate to form. The mixture was made basic and filtered. The resulting pasty solid was added to benzene, and the benzene and remaining water were evaporated. The tan solid was dried in a vacuum oven at 110°C, yielding 7.2g. This product was added to about 700 ml absolute ethanol. After stirring and heating, the insoluble sodium sulfate was filtered off and the ethanol was evaporated yielding 4.0g alkylbenzene sulfonate. Liquid chromatography showed this product to contain less than 10% sodium sulfate. This material was submitted for biodegradation testing without further purification and was found to be biodegradable. More particularly, the alkylbenzene sulfonate made with the present oligomers (nearly linear) was as biodegradable as an alyklbenzene sulfonate prepared from 1-dodecene (linear) and using $AlCl_3$ as the alkylation catalyst. In this test microbial cultures were acclimated to surfactants at least three weeks prior to tests. The EPA Shake Flask Method was used. This method is described at Federal Register V. 44(53), A-5.1, March 16, 1979, pp. 16274-16275. Results are summarized in Table 6.

Table 6

| ALKYLBENZENE SULFONATE ULTIMATE BIODEGRADABILITY | | |
|---|---|---|
| $C_{12}$ Olefin Structure | Alkylation Catalyst | % of Test Compound Converted to $CO_2$ (8 days) |
| Linear | $AlCl_3$ | 27 |
| Nearly Linear | Dealuminized Mordenite | 25 |

**Claims**

1. A process for oligomerizing an olefin feedstock comprising the step of contacting the feedstock with ZSM-23 which is surface deactivated with a trialkyl pyridine compound having an effective cross-section larger than that of the pores of ZSM-23.

2. The process of claim 1 wherein surface deactivation is with a trialkylpyridine compound wherein the alkyl groups have 1-4 carbon atoms and are at the 2, 4 and 6 positions on the pyridine ring.

3. The process of claim 2 wherein the trialkylpyridine compound is 2, 4, 6 collidine.

4. The process of any preceding claim wherein the olefin feedstock comprises propylene and/or butene.

5. The process of any preceding claim wherein said contacting step is effected at a temperature of 100-350°C, a pressure of atmospheric to 20,000 kPa and a WHSV of 0.01-2.

6. The process of any preceding claim wherein said contacting step is effected to a temperature of 150-250°C, a pressure of 1000-10,000 kPa and a WHSV of 0.2-1.

7. A method of producing a lubricating oil comprising the steps of
   a) oligomerizing a $C_3$ and/or $C_4$ olefin feedstock by a process as claimed in any preceding claim; and
   b) oligomerizing the $C_{12+}$ fraction of the product of step (a).

8. A method of producing a biodegradable alkylbenzene sulfonate comprising the steps of:

12

a) oligomerizing a $C_3$ and/or $C_4$ olefin feedstock by a process as claimed in any one of claims 1 to 6;

b) alkylating benzene with the $C_{12}$ fraction of the product of step (a); and

c) sulfonating the product of step (b).

**Revendications**

1. Un procédé d'oligomérisation d'une charge d'oléfines comprenant l'étape de contact de la charge avec une ZSM-23 désactivée en surface par un dérivé de trialkvlpyridine et présentant une section transversale efficace supérieure à celle des pores de la ZSM-23.

2. Le procédé selon la revendication 1, caractérisé en ce que la désactivation superficielle est faite à l'aide d'un dérivé de trialkylpyridine dans lequel les groupes alkyles renferment de 1 à 4 atomes de carbone et sont situés dans les positions 2, 4 et 6 du cycle de la pyridine.

3. Le procédé selon la revendication 2, caractérisé en ce que la trialkylpyridine est la 2,4,6-collidine.

4. Le procédé selon l'une quelconque des revendications précédentes, caractérisé en ce que la charge d'oléfines comprend du propylène et/ou du butène.

5. Le procédé selon l'une quelconque des revendications précédentes, caractérisé en ce que cette étape de contact est effectuée à une température de 100 à 350°C, une pression comprise entre la pression atmosphérique et 20 000 kPa et une VSHP de 0,01 à 2.

6. Le procédé selon l'une quelconque des revendications précédentes, caractérisé en ce que cette étape de contact est effectuée à une température de 150 à 250°C, une pression de 1 000 à 10 000 kPa et une VSHP de 0,2 à 1.

7. Un procédé de préparation d'une huile lubrifiante comprenant les étapes suivantes:
   a) on oligomérise une charge d'oléfines en $C_3$ et/ou $C_4$ par un procédé selon l'une quelconque des revendications précédentes; et
   b) on oligomérise la fraction en $C_{12}$ + du produit obtenu dans l'étape (a).

8. Un procédé de préparation d'un alkylbenzène sulfonate biodégradable comprenant les étapes suivantes:
   a) on oligomérise une charge d'oléfines en $C_3$ et/ou $C_4$ + par un procédé selon l'une quelconque des revendications 1 à 6;
   b) on alkyle du benzène par la fraction $C_{12}$ du produit de l'étape (a): et
   c) on sulfone le produit de l'étape (b).

**Patentansprüche**

1. Verfahren zur Oligomerisation eines Olefinausgangsmaterials, das den Schritt des Kontakts dieses Ausgangsmaterials mit ZSM-23 umfaßt, dessen Oberfläche mit einer Trialkylpyridinverbindung deaktiviert wurde, die einen wirksamen Querschnitt aufweist, der größer als der der Poren des ZSM-23 ist.

2. Verfahren nach Anspruch 1, worin die Deaktivierung der Oberfläche mit einer Trialkylpyridinverbindung erfolgt, worin die Alkylgruppen 1-4 Kohlenstoffatome aufweisen und in der 2-, 4- und 6-Position am Pyridinring sind.

3. Verfahren nach Anspruch 2, worin die Trialkylpyridinverbindung 2,4,6-Collidin ist.

4. Verfahren nach einem der vorstehenden Ansprüche, worin das Olefinausgangsmaterial Propylen und/oder Buten umfaßt.

5. Verfahren nach einem der vorstehenden Ansprüche, worin der Schritt des Kontaktes bei einer Temperatur von 100-350°C, einem Druck von atmosphärischem Druck bis 20.000 kPa und einer WHSV von 0,01-2 durchgeführt wird.

13

**6.** Verfahren nach einem der vorsteheden Ansprüche, worin der Schritt des Kontaktes bis zu einer Temperatur von 150-250°C, einem Druck von 1000-10.000 kPa und einer WHSV von 0,2-1 durchgeführt wird.

**7.** Verfahren zur Herstellung von Schmieröl, welches die Schritte umfaßt:
a) Oligomerisation einer $C_3$- und/oder $C_4$-Olefin-Zufuhr durch ein Verfahren nach einem der vorstehenden Ansprüche und
b) Oligomerisation der $C_{12+}$-Fraktion des Produktes vom Schritt (a).

**8.** Verfahren zur Herstellung von biologisch abbaubarem Alkylbenzolsolfonat, welches die Schritte umfaßt:
a) Oligomerisation einer $C_3$- und/oder $C_4$-Olefinzufuhr durch ein Verfahren nach einem der Ansprüche 1 bis 6,
b) Alkylierung von Benzol mit der $C_{12}$-Fraktion des Produktes vom Schritt (a) und
c) Sulfonierung des Produktes vom Schritt (b).